# EUROPEAN PATENT APPLICATION

(11) **EP 4 498 084 A1**
(43) Date of publication of application: **29.01.2025**
(21) Application number: 22933582.3
(22) Date of filing: 24.08.2022
(51) Int. Cl.: G01N 33/50, G01N 33/52, G01N 27/62

(54) **METHOD FOR QUANTIFYING NICOTINAMIDE ADENINE DINUCLEOTIDE (NAD+) OR NICOTINAMIDE MONONUCLEOTIDE (NMN), AND KIT AND PAPER FILTER FOR PERFORMING SAID METHOD**

(30) Priority: 23.03.2022 JP 2022046509
(71) Applicant: Teijin Limited, Osaka 530-0005 (JP)
(72) Inventor: MATSUYAMA Ryo, Osaka-shi Osaka 530-0005 (JP); OMATA Tomoyo, Tokyo 100-0013 (JP)
(74) Representative: Carpmaels & Ransford LLP
(86) International application number: PCT/JP2022/031851
(87) International publication number: WO 2023/181451

(57) **Abstract**

[Problem]

To provide an accurate quantitative method using the DBS method for NAD+ or NAD+ precursors such as NMN.

[Solution]

A method for quantifying nicotinamide adenine dinucleotide (NAD+) or nicotinamide mononucleotide (NMN), comprising Step 1 of bringing a filter paper, impregnated with a radical inhibitor or a chaotropic agent, and with added blood derived from a test subject, into contact with a solvent; and Step 2 of quantifying the nicotinamide adenine dinucleotide (NAD+) or nicotinamide mononucleotide (NMN) in the solvent obtained in Step 1.

## Description

### [Technical Field]

The present invention relates to a method for quantifying nicotinamide adenine dinucleotide (NAD+) or nicotinamide mononucleotide (NMN), and a kit and filter paper for performing the method.

### [Background Art]

Nicotinamide adenine dinucleotide (NAD+) is a coenzyme essential for energy-yielding reactions. Furthermore, the NAD+ has been reported to decrease with age in rodents, humans, etc. (NPL 1).

In contrast, nicotinamide mononucleotide (NMN) is also present in various biological species and, together with nicotinamide riboside (NR) etc., collectively referred to as the vitamin B3 group which is a precursor of NAD+. Further, these NAD+ precursors are reported to enable rodents and the like to improve the pathology of agingrelated diseases by increasing NAD+ in vivo (NPL 2). In addition, increasing NAD+ levels in vivo recently has been attracting attention as seen in selling anti-aging supplements of NAD+ precursors such as NMN.

Therefore, quantifying the in-vivo levels of the above-mentioned NAD+ and NAD+ precursors such as NMN expectably leads to understanding the pathological conditions and degree of aging. Accordingly, various methods are under investigation to quantify the level of NAD+ and NAD+ precursors such as NMN. For example, NPL 3 reports that NAD+ is detectable by the dried blood spot method (DBS method).

Here, the above-mentioned DBS method is a method to quantify the concentration of the measuring target substance present in the blood derived from a test subject by dropping (or spotting) the blood onto a special filter paper, drying it, and then performing an extraction operation. The DBS method gives the following merits: (1) using blood as is can save an operation of separating plasma components from blood, (2) a required amount of blood smaller than conventional blood tests enables self-collection, and (3) using filter paper allows easy delivery to testing institutions, etc. Therefore, the method is actually used in newborn mass screening, therapeutic drug monitoring (TDM) at remote location, etc., and is attracting attention.

### [Citation List]

### [Non Patent Literature]

[NPL 1] Experimental gerontology, 2020, 134, 110888
[NPL 2] Biochemistry, Vol. 87, No. 2, p. 239-244, 2015
[NPL 3] Metabolites, 2017, 7, 35

### [Summary of Invention]

### [Technical Problem]

Quantifying the above-mentioned NAD+ and NAD+ precursors such as NMN by the DBS method described in NPL 3 still has a problem of inaccurate quantification. This is because of the following reasons: Firstly, the storage conditions tend to alter greatly the degree of decomposition of NAD+ and NAD+ precursors such as NMN, which requires a special processing beforehand such as quick freezing for the quantification; further, the DBS method, described in NPL 3 etc., has poor stability, such as storage stability after dropping onto a filter paper, depending on the quantifying target substance, which may disturb accurate quantification.

An object of the present invention is to provide an accurate quantitative method using the DBS method for NAD+ or NAD+ precursors such as NMN.

### [Solution to Problem]

The present invention was made for the purpose of solving the above problems, and consists of the following items.
(1) A method for quantifying nicotinamide adenine dinucleotide (NAD+) or nicotinamide mononucleotide (NMN) in a test subject, the method comprising the following Steps 1 and 2:
   Step 1 of bringing a filter paper, impregnated with a radical inhibitor or a chaotropic agent, and with added blood derived from the test subject, into contact with a solvent;
   Step 2 of quantifying the nicotinamide adenine dinucleotide (NAD+) or nicotinamide mononucleotide (NMN) in the solvent obtained in Step 1.
(2) The method according to (1), wherein the method is for quantifying nicotinamide adenine dinucleotide (NAD+).
(3) The method according to (1) or (2), wherein the filter paper is impregnated with a chaotropic agent.
(4) The method according to (1) or (2), wherein the radical inhibitor contains a surfactant.
(5) The method according to (4), wherein the surfactant is an anionic surfactant.
(6) The method according to (5), wherein the anionic surfactant is sodium dodecyl sulfate.
(7) The method according to (3), wherein the chaotropic agent contains a guanidine salt.
(8) The method according to (7), wherein the guanidine salt is guanidine thiocyanate.
(9) The method according to any one of (1) to (8), wherein the filter paper is made of cellulose.
(10) The method according to any one of (1) to (9), wherein the solvent is water and/or acetonitrile.
(11) The method according to (10), wherein the solvent is water.
(12) The method according to any one of (1) to (11), wherein the quantifying in Step 2 is performed by a method using a mass spectrometer or a colorimetric method.
(13) A kit for performing the method according to any one of (1) to (12), comprising: a blood sampling device and a filter paper impregnated with a radical inhibitor or a chaotropic agent.
(14) A filter paper impregnated with a radical inhibitor or chaotropic agent for performing the method according to any one of (1) to (12).

### [Advantageous Effects of Invention]

The present invention provides good storage stability for NAD+ or NAD+ precursors such as NMN, and thus provides an accurate quantitative method using the DBS method for NAD+ or NAD+ precursors such as NMN.

### [Description of Embodiments]

### Method for quantifying nicotinamide adenine dinucleotide (NAD+) or nicotinamide mononucleotide (NMN)

The method of the present invention for quantifying nicotinamide adenine dinucleotide (NAD+) or nicotinamide mononucleotide (NMN) (hereinafter may be abbreviated as the quantitative method of the present invention) is comprising the following steps: Step 1 (hereinafter may be abbreviated as Step 1 of the present invention) of bringing a filter paper, impregnated with a radical inhibitor or a chaotropic agent, and with added blood derived from a test subject, into contact with a solvent; and Step 2 (hereinafter may be abbreviated as Step 2 of the present invention) of quantifying the NAD+ or NMN in the solvent obtained in Step 1.

The details of the quantitative method of the present invention will be described below.

### Nicotinamide adenine dinucleotide (NAD+)

In the quantitative method of the present invention, nicotinamide adenine dinucleotide (NAD+) is, as described above, a coenzyme essential for energy-yielding reactions. It is also known as an electron transport system in all eukaryotes, archaebacteria, eubacteria, etc.

Note that nicotinamide adenine dinucleotide can take two states of oxidized and reduced in vivo, and refers to, in the present invention, oxidized nicotinamide adenine dinucleotide (i.e., NAD+). In addition, old names meaning NAD+, such as diphosphopyridine nucleotide, coenzyme I, and codehydrogenase I, are also included in nicotinamide adenine dinucleotide in the present invention.

### Nicotinamide mononucleotide (NMN)

In the quantitative method of the present invention, nicotinamide mononucleotide (NMN), as described above, is an NAD+ precursor that is present in various biological species and is collectively referred to as the vitamin B3 group. NMN is also known to have various functions, such as activation of mitochondria and activation of sirtuin genes, so-called longevity genes.

Note that the structure of NMN is as follows.

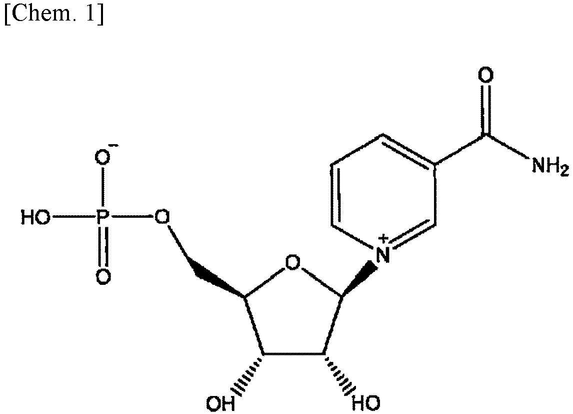

As described above, the quantifying target substance in the quantitative method of the present invention is NAD+ and/or NMN, and the quantitative method of the present invention allows accurate quantification of them. Among them, based on the viewpoint of accuracy of quantification, NAD+ is more preferable as the quantifying target substance in the quantitative method of the present invention.

Needless to say, the above-mentioned "quantification" in the present invention refers to calculating the concentration, mass, molar amount, etc. of NAD+ or NMN in blood derived from a test subject as described later.

### Test subject

Examples of test subjects in the quantitative method of the present invention include mammals such as humans, monkeys, mice, rats, dogs, cats, pigs, and rabbits, preferably humans, monkeys, mice, and rats, and more preferably humans. In addition, such humans include, for example, healthy individuals, patients suffering from diseases associated with increase or decrease in NAD+ and/or NMN, and patients suspected to suffer from diseases associated with increase or decrease in NAD+ and/or NMN. Specific examples of such diseases include, for example, age-related diseases.

### Step 1

Step 1 of the present invention is a step of bringing a filter paper, impregnated with a radical inhibitor or a chaotropic agent, and with added blood derived from a test subject, into contact with a solvent. This Step 1 will be explained in detail as follows.

The blood in Step 1 of the present invention refers to blood derived from the above-mentioned test subject, and specifically includes, for example, whole blood, blood cells, plasma, and serum, among which whole blood is preferable.

In addition, the method for sampling blood derived from a test subject may be any method commonly used in this field, such as a method using a self-blood sampling device. Examples of such self-blood sampling devices include Micro Blood Collection Kit (Micro Blood Science Co., Ltd.). When the test subject is a human, blood can be sampled, for example, from the human's fingertips or toes using the self-blood sampling device described above.

The filter paper in Step 1 of the present invention is subject matter to which blood derived from the test subject is added, and is impregnated with a radical inhibitor or chaotropic agent. Among these, filter paper impregnated with a chaotropic agent is more preferable from the viewpoint of accuracy of quantification.

The above-mentioned radical inhibitor is a substance that destroys proteinprotein interaction and denatures proteins, and specifically includes those containing, for example, a surfactant and a buffer.

Examples of such surfactants include anionic surfactants, cationic surfactants, ampholytic surfactants, nonionic surfactants, among which anionic surfactants are preferable. Examples of such anionic surfactants include sodium dodecyl sulfate (may be referred to as sodium lauryl sulfate), lithium dodecyl sulfate, sodium cholate, and sodium deoxycholate, among which sodium dodecyl sulfate is preferable.

Examples of such buffers include Tris buffers such as tris(hydroxymethyl)aminomethane buffers, and Good's buffers such as phosphate buffers, citrate buffers, glycine buffers, 3-morpholinopropanesulfonic acid buffers, acetate buffers, veronal buffers, borate buffers, and carbonate buffers, among which Tris buffers are preferable.

The above-mentioned chaotropic agent is a substance that reduces the interaction between water molecules and thereby destabilizes the structure of a protein, and contains at least a guanidine salt.

Examples of such guanidine salts include guanidine thiocyanate, guanidine hydrochloride, guanidine nitrate, and guanidine sulfate, among which guanidine thiocyanate is preferable.

The method for impregnating the filter paper with the radical inhibitor and chaotropic agent can be any method as long as commonly used in this field, and examples thereof include vacuum impregnation and a vacuum pressure impregnation method to immerse filter paper in a solution containing the radical inhibitor or chaotropic agent for a certain period of time, and a method of direct application or coat of the radical inhibitor or chaotropic agent on the filter paper.

The main component (base) of the filter paper in Step 1 of the present invention includes cellulose, cotton, and pulp, among which cellulose is preferable. The method for obtaining such cellulose-based filter paper may be any method as long as commonly used in this field. Such a method is, for example, as follows: water and cellulose are mixed, water is removed from the mixture using a paper machine, the mixture is dried, and then a filter paper is produced using an automatic filter paper punching machine. Further, the method described in Japanese Examined Patent Application Publication No. 08-032995 can also be referred to.

The thickness of the filter paper in Step 1 of the present invention may be in the range of 500 µm to 800 µm, and preferably in the range of 550 µm to 750 µm.

The weight of the filter paper in Step 1 of the present invention may be, for example, weight per unit area (mg/cm²) of 5 mg/cm² to 50 mg/cm², and preferably 15 mg/cm² to 35 mg/cm².
In particular, when the filter paper in Step 1 of the present invention is impregnated with a radical inhibitor, the weight of the filter paper may be, for example, weight per unit area (mg/cm²) of 5 mg/cm² to 50 mg/cm², and preferably 15 mg/cm² to 20 mg/cm². Further, when the filter paper in Step 1 of the present invention is impregnated with a chaotropic agent, the weight of the filter paper may be, for example, weight per unit area (mg/cm²) of 5 mg/cm² to 50 mg/cm², and preferably 30 mg/cm² to 35 mg/cm².

Note that, when measuring the weight per unit area, for example, a Sartorius electronic balance (MSA225S-100-DI) can be used.

In addition, for the filter paper in Step 1 of the present invention, other parameters such as length and width, shape, impregnation concentration of the radical inhibitor and chaotropic agent, etc., can be set optionally considering the actual use conditions, etc. In particular, such shapes include, though not limited thereto, circular shapes and square shapes.

The filter paper in Step 1 of the present invention may be any paper as long as having the above-mentioned characteristics, and examples of filter paper satisfying such characteristics include Whatman FTA DMPK-A Card (GE Healthcare), QIAcard FTA DMPK-A Card (QIAGEN), Whatman FTA DMPK-B Card (GE Healthcare), and QIAcard FTA DMPK-B Card (QIAGEN), which may be used.

Note that, the above-mentioned Whatman FTA DMPK-A Card (GE Healthcare) and QIAcard FTA DMPK-A Card (QIAGEN) are produced by impregnating with a radical inhibitor (sodium dodecyl sulfate) as described in the Examples below, and the main component of the filter paper is cellulose. Further, the above-mentioned Whatman FTA DMPK-B Card (GE Healthcare) and QIAcard FTA DMPK-B Card (QIAGEN) are produced by impregnating with a chaotropic agent (guanidine thiocyanate) as described in the Examples below, and the main component of the filter paper is cellulose.

The solvent in Step 1 of the present invention is a solvent for extracting components including NAD+ and NMN from a filter paper with added blood derived from a test subject, and specifically, examples thereof include water, acetonitrile, a mixture of water and acetonitrile, and alcohols such as ethanol, among which water or a mixture of water and acetonitrile is preferable, and water is more preferable. Examples of such water include ion-exchanged water, deionized water, pure water, ultrapure water, and distilled water, among which ultrapure water is preferable. Further, when such a mixture of water and acetonitrile is used, the ratio of the two may be in the range of 80% water : 20% acetonitrile to 20% water : 80% acetonitrile, and preferably in the range of 80% water : 20% acetonitrile to 40% water : 60% acetonitrile.

In addition, when preparing the above-mentioned solvent, an internal standard substance depending on the quantifying target substance may be added to the solvent.

In Step 1 of the present invention, the method of bringing the filter paper into contact with the solvent may be any method as long as commonly used in this field, and specifically, examples thereof include a method of adding a solvent to a filter paper at such an amount as to fully soak the filter paper and mixing it. Such an amount is, for example, 100 µL to 1000 µL, and can be appropriately adjusted depending on the conditions of the filter paper and the performance of the equipment etc. used for quantification as described below.

More specifically, the above method may be performed as follows, for example. First, a certain amount of blood derived from a test subject is sampled in advance from the test subject, and 1 µL to 10 µL of the blood is spotted on a predetermined location on a filter paper. Thereafter, the blood-spotted area on the filter paper is cut out and put into a tube such as a 2 mL tube. Next, 100 µL to 1000 µL of the above solvent is added to the tube. After adding the solvent, the mixture may optionally be mixed for a certain period of time (about 10 minutes to 60 minutes) at room temperature using a mixer such as a micro mixer.

In addition, when performing the above method, if necessary, methods described in the following documents may be referred to: NPL 1, NPL 3, JP-T-2015-508493, JP-T-2018-530766, JP-T-2014-503197, JP-T-2011-506922, JP-T-2004-505277, Uniformity of Dry Matrix Spot, Application Note (Agilent Technologies, Inc.), and the like.

As described above, in Step 1 of the present invention, bringing the filter paper with added blood derived from the test subject into contact with the solvent allows the components including NAD+ and NMN to be extracted from the filter paper with added blood derived from the test subject.

In addition, after performing Step 1 of the present invention and before performing Step 2 of the present invention described below, in order to stabilize the quantifying target substance in the solvent obtained in Step 1 of the present invention, a step of adding a certain amount of organic solvent may be performed. Examples of such organic solvents include acetonitrile, methanol, and ethanol, among which acetonitrile is preferable. Further, the addition amount of the organic solvent may be, for example, twice the amount of the solvent 1 in Step 1 of the present invention.

### Step 2

Step 2 of the present invention is a step of quantifying NAD+ or NMN in the solvent obtained by Step 1 of the present invention. Hereinafter, this Step 2 will be explained in detail.

The method for quantifying NAD+ or NMN in Step 2 of the present invention is a method for quantifying NAD+ or NMN present in the solvent obtained by the operation in Step 1 of the present invention and may be any method as long as commonly used in this field, and examples thereof include a method using a mass spectrometer, a colorimetric method, and a fluorescence method.

Methods using such mass spectrometers include a method using liquid chromatograph mass spectrometer (LC-MS/MS), gas chromatograph mass spectrometer (GC/MS), or capillary electrophoresis mass spectrometer (CE/MS). Specifically, in these methods, components in a biological sample (blood, etc.) may be separated in a separation section having a chromatograph such as a liquid chromatograph, and the various separated components are ionized in a mass spectrometry section, and then separated to each massto-charge ratio (m/z). More specifically, method may be performed by referring to the documents attached to the device or the methods described in, for example, WO2014/038524, WO2017/19588, and WO2018/034346.

Such a colorimetric method is a method for quantifying a target substance based on the degree of color development obtained by a reaction of a biological sample (blood, etc.) and a coloring agent, using a microplate reader such as a microplate spectrophotometer.

Such a coloring agent may be any one as long as commonly used in this field, and specific examples thereof include orthocresol phthalein complexone (OCPC), arsenazo-III, chlorophosphonazo-III, and eriochrome black T, glyoxal-bis(2-hydroxyanil), NN (2-hydroxy-1-(2-hydroxy-4'-sulfo-1'-naphthylazo)-3-naphthoic acid), and hydroxynaphthol blue. Specifically, the above-mentioned colorimetric method may be performed in accordance with the method commonly used in this field, for example, with reference to JP-A No. 2003-262629.

In addition, quantification by the colorimetric method described above may be performed using a commercially available kit, and specific examples of such a kit include NAD/NADH Assay Kit-WST (manufactured by Dojindo Laboratories Co., Ltd.), NAD+/NADH Assay Kit (Colorimetric) (CELL BIOLABS, INC.).

Such a fluorescence method is a method that is performed by irradiating light onto a biological sample (such as blood) containing a target substance and detecting the light generated when the excited substance returns to the ground state, and specifically, a method for quantifying a target substance based on the intensity of light generated when excited electrons return to the ground state, using a plate reader such as a fluorescence plate reader. Specifically, the above-mentioned fluorescence method may be performed in accordance with the method commonly used in this field, for example, with reference to JP-A No. 2012-202742, and JP-A No. 2009-276162.

In addition, quantification by the above-mentioned fluorescence method may be performed using a commercially available kit, and specific examples of such a kit include NAD+/NADH Assay Kit (Fluorometric) (CELL BIOLABS, INC.).

### Kit for performing the method for quantifying NAD+ or NMN

A kit for performing the method for quantifying NAD+ or NMN of the present invention (hereinafter may be abbreviated as a kit of the present invention) includes a blood sampling device and filter paper impregnated with a radical inhibitor or a chaotropic agent.

The kit of the present invention will be described in detail below.

Such a blood sampling device is for collecting blood from a test subject, and is the same as described in quantitative method of the present invention, which applies herein to specific examples, preferred examples, etc.

Such a filter paper is impregnated with a radical inhibitor or chaotropic agent, and is the same as explained in quantitative method of the present invention, which applies herein to specific examples, preferred examples, etc.

The kit of the present invention includes at least the blood sampling device described above and a filter paper impregnated with a radical inhibitor or a chaotropic agent, and may optionally include instructions, a container for storing the filter paper, etc.

Such instructions practically describe the principles, operating procedures, etc. of the quantitative method of the present invention in the form of text, drawings, etc., and forms thereof include instruction manuals, attached documents, pamphlets, and leaflets.

Such a container may be any one as long as capable of storing the filter paper, and preferably having the function of absorbing moisture. Specific examples of such containers include, for example, moisture absorption standard pouch. Specific examples of such moisture absorption standard pouch include Moisture Absorption-kun (Maruto Sangyo Co., Ltd.) (registered trademark).

The kit of the present invention allows the quantitative method of the present invention to be performed more simply, more quickly, and more accurately. Further, the kit of the present invention allows a test subject (e.g., a human) at a specific place such as the home to prepare a filter paper with added blood derived from the test subject, and mail the filter paper to an institution (e.g., testing institutions, medical institutions, etc. having mass spectrometers such as LC-MS/MS etc.), and the institution to quantify NAD+ and NMN, and further, if necessary, integrated services to be provided such as feeding back the quantitative results to the test subject. As shown in the examples described above and below, the filter paper included in the kit of the present invention has such excellent storage stability of NAD+ and NMN to be fully applicable also to the above service etc. requiring the filter paper to be mailed.

### Application of the quantitative method of the present invention

The above-mentioned quantitative method of the present invention also has applicability to various fields. For example, as mentioned above, age-related diseases, etc. are known as diseases associated with increase and decrease in NAD+ and NMN. Therefore, presumably enabled is an analysis (e.g., determination, diagnosis, etc.) using the quantitative results obtained by the quantitative method of the present invention and the relationship thereof with the above-mentioned diseases.

### [Examples]

Hereinafter, the present invention will be explained in more detail with reference to Examples, but the scope of the present invention is not limited thereby.

### Example 1: Optimization study of extraction buffer

Searched for was an optimal solvent (i.e., This corresponds to the solvent in Step 1 of the present invention. Hereinafter, it may be abbreviated as extraction buffer.) used to extract components including NAD+ and NMN from a specific filter paper with added blood derived from a test subject.

Note that, to add explanation to the purpose of Example 1, the focus is on optimizing the above-mentioned extraction buffer in Example 1, and thus no filter paper is used. The specific method is explained by a following experimental system: Various candidate extraction buffers are added to blood, and the resulting solutions are used as they are for quantification of NAD+ and NMN using LC-MS/MS.

### Experimental method

Six-week-old male SD rats (3 rats) were subjected to laparotomy under isoflurane inhalation anesthesia, and whole blood was collected from the abdominal aorta. After dispensing 200 µL of the collected blood (i.e., whole blood) from each individual into a 1.5 mL microtube, centrifugation (13,200 ×g, 5 minutes, 4°C) was performed. The supernatant after centrifugation was removed, and the centrifuged residue was used as a blood cell fraction.

After that, ultrapure water (Milli-Q integral 5 system/Merck Ltd.) and acetonitrile (Thermo Fisher Scientific) were mixed respectively at ratios of 100:0, 80:20, 60:40, 40:60, 20:80 and 0:100, to prepare six types of extraction buffers.

To a 1.5 mL microtube added are 5 µL of blood or blood cells of each individual, 10 µL of a mixed aqueous solution of internal standard substance (NAD+-d4 and NMN-d5/Toronto Research Chemicals, Inc.), and 295 µL of extraction buffer, then the mixture was stirred with a vortex mixer, and the mixture was mixed at room temperature for 30 minutes using a micromixer (TOMY MICRO TUBE MIXER MT-400, stirring speed 5). The extract solution of blood or blood cell after mixing was dispensed by each 100 µL portion into a microtube, 200 µL of acetonitrile was added thereto, and the mixture was stirred using a vortex mixer, followed by centrifugation (24,250 ×g, 10 minutes, 4°C). The supernatant after centrifugation was dispensed by 100 µL into a vial, and a mixed aqueous solution of an internal standard substance at a dilution depending on the extraction buffer used was added and mixed at an amount of 0 µL to 50 µL so that the final organic solvent content was 67%. Thereafter, NAD+ and NMN were each measured by LC-MS/MS in accordance with the following analysis conditions.

### LC-MS/MS measurement conditions

**[Table 1]**

| | | |
|---|---|---|
| Device | MS | Triple quadrupole mass spectrometer 4000 QTRAP (AB Sciex) |
| | LC | Ultra-high performance liquid chromatograph Nexera X2 (Shimadzu Corporation) |
| Column | | Atlantis Hilic (4.6 mm × 50 mm, 5 µm) (Waters) |
| Column temperature | | 50°C |
| Injection amount | | 5 µL |
| Flow rate | | 1.0 mL/min |
| Auto-sampler temperature | | 4°C |
| Mobile phase A | | 10 mM ammonium formate water containing 0.05% formic acid |
| Mobile phase B | | Acetonitrile |
| Washings 1 | | Acetonitrile : water (9:1, v/v) |
| Washings 2 | | Acetonitrile : Mobile phase A (97:3, v/v) |
| Ion Source | | Turbo Spray |
| Polarity | | Positive |

**[Table 2]**

| | Time (min) | B (%) |
|---|---|---|
| Gradient conditions | Initial | 97 |
| | 5.00 | 40 |
| | 7.00 | 40 |
| | 7.10 | 97 |
| | 10.00 | 97 |

**[Table 3]**

| | Compound | Q1 | Q3 |
|---|---|---|---|
| Q1/Q3 | NAD | 664.056 | 136.1 |
| | NAD-d4 | 668.198 | 136.1 |
| | NMN | 335.098 | 123.1 |
| | NMN-d5 | 340.125 | 127.0 |

### Experimental result

The above experimental results are shown in Table 4.

**[Table 4]**

| Quantifying target substance | Extraction buffer composition (Water: Acetonitrile) | Concentration (µmol/L) | | | | Extraction efficiency (%) | | | | | |
|---|---|---|---|---|---|---|---|---|---|---|---|
| | | Blood | | Blood cells | | Blood | | | Blood cells | | |
| | | Mean | Standard deviation | Mean | Standard deviation | Mean | Standard deviation | Evaluation | Mean | Standard deviation | Evaluation |
| NAD+ (Lower limit of quantification: 1 µM) | 0 : 100 | NC | NC | NC | NC | NC | NC | Poor | NC | NC | Poor |
| | 20 : 80 | 18.28 | 3.89 | 31.53 | 6.00 | 9.4 | 3.0 | Poor | 9.1 | 3.6 | Poor |
| | 40 : 60 | 124.05 | 13.98 | 203.81 | 33.78 | 62.5 | 5.1 | Good | 55.9 | 12.1 | Average |
| | 40 : 40 | 141.35 | 10.45 | 212.89 | 26.45 | 71.8 | 12.0 | Good | 58.8 | 14.0 | Average |
| | 80 : 20 | 135.60 | 25.70 | 232.44 | 22.34 | 68.0 | 10.3 | Good | 64.3 | 14.6 | Good |
| | 100 : 0 | 200.78 | 40.22 | 380.82 | 130.19 | 100.0 | 0.0 | Excellent | 100.0 | 0.0 | Excellent |
| NMN (Lower limit of quantification: 2 µM) | 0 : 100 | NC | NC | NC | NC | NC | NC | Poor | NC | NC | Poor |
| | 20 : 80 | NC | NC | NC | NC | NC | NC | Poor | NC | NC | Poor |
| | 40 : 60 | 5.54 | 1.53 | 9.96 | 0.76 | 85.4 | 21.1 | Excellent | 83.0 | 9.3 | Excellent |
| | 60 : 40 | 5.88 | 0.83 | 12.58 | 0.92 | 91.1 | 11.4 | Excellent | 104.9 | 11.2 | Excellent |
| | 80 : 20 | 6.25 | 1.20 | 12.23 | 1.03 | 96.5 | 15.8 | Excellent | 101.7 | 5.6 | Excellent |
| | 100 : 0 | 6.45 | 0.19 | 12.03 | 0.65 | 100.0 | 0.0 | Excellent | 100.0 | 0.0 | Excellent |
| *Extraction efficiency = concentration ratio for each extraction buffer, assuming the concentration when using extraction buffer (water : acetonitrile = 100:0) as 100% 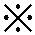 | | | | | | | | | | | |
| *Evaluation = Excellent (≥80%), Good (≥60%), Average (≥ 40%), Poor (<40%) NC= Not Calculated | | | | | | | | | | | |

As is clear from Table 4, among six types of extraction buffers used with different acetonitrile content ratios, regarding NAD+ and NMN in blood, the highest concentration was exhibited by the extraction buffer only using water as an extraction buffer (i.e., water = 100% and acetonitrile content = 0%). In addition, the extraction buffer prepared to have an acetonitrile content of about 60%, though exhibiting concentrations of NAD+ and NMN lower than the case of an acetonitrile content of 0% (i.e., only water), was confirmed to exhibit certain concentrations of NAD+ and NMN.

However, the extraction buffer prepared to have an acetonitrile content of 80% or more was found to exhibit greatly decreased concentrations of NAD+ and NMN. In addition, the same tendency of the result was also shown in the case of using blood cells.

From the result of Example 1, water was found to be the most efficient solvent to extract NAD+ and NMN from blood in their quantification in blood.

Considering this result, the quantifying targets of NAD+ and NMN are present in blood cells; therefore, the osmotic pressure action by water inferably destroyed the blood cell components, resulting in their efficient detection.

### Example 2: Optimization study of filter paper based on the viewpoint of chilled storage stability

A filter paper, to which blood derived from a test subject is added (i.e., corresponding to a filter paper in Step 1 of the present invention), was searched for the optimal one for quantifying NAD+ and NMN when the filter paper with added blood derived from the test subject is stored in the refrigerator for a certain period of time.

The specific experimental method is as follows.

### Experimental method

From 5 subjects (adult men and women) per test, approximately 120 µL of blood (amount of 2 collection containers of the kit) was sampled by self-collection using an Micro Blood Collection Kit (Micro Blood Science Co., Ltd.), and was left to stand on ice until sample processing. The subjects were fasted on the morning of the sampling day, and samplings were performed around 9:00 am. The test was performed four times in total, with a total of 19 people performing evaluations, excluding one person due to insufficient sample blood volume.

The sampled blood was collected into 1.5 mL microtubes for each subject and mixed by gentle pipetting. Four types of filter papers (Whatman FTA DMPK-A Card/GE healthcare, QIAcard FTA DMPK-B Card/QIAGEN, QIAcard FTA DMPK-C Card/QIAGEN, Whatman 903 Protein saver card/Cytiva) were each spotted with 5 µL of blood (dropped/added) at multiple areas, sealed in a moisture absorption standard pouch (Moisture absorption-kun (registered trademark) / Maruto Sangyo Co., Ltd.), and stored in the refrigerator. Next, after chilled storing the blood-spotted filter paper for 2 days, 2 weeks (14 days or 15 days), or 1 month (30 days), the blood-spotted areas were cut out and collected in a 2 mL microtube. After adding 300 µL of a mixed aqueous solution of internal standard substances (NAD+-d4 and NMN-d4/Toronto Research Chemicals, Inc.) thereto and stirring using a vortex mixer, the mixture was mixed with a micromixer (TOMY MICRO TUBE MIXER MT-400, stirring speed 5) for 30 minutes at room temperature. The blood extract solution was dispensed by each 150 µL portion into a microtube, 300 µL of acetonitrile was added thereto, and the mixture was stirred using a vortex mixer, followed by centrifugation (24,250 ×g, 10 minutes, 4°C). After centrifugation, 40 µL of the supernatant was dispensed into a vial and measured by LC-MS/MS.

In contrast, as a sample for initial values, 5 µL of blood was collected into a 2 mL microtube (no spotted filter paper), and 300 µL of a mixed aqueous solution of internal standard substances (NAD-d4 and NMN-d4/Toronto Research Chemicals, Inc.) was added thereto and stirred using a vortex mixer, and then the mixture was mixed using a micromixer (TOMY MICRO TUBE MIXER MT-400, stirring speed 5) for 30 minutes at room temperature. The blood extract solution was dispensed by each 150 µL portion into a microtube, 300 µL of acetonitrile was added thereto, and the mixture was stirred using a vortex mixer, followed by centrifugation (24,250 ×g, 10 minutes, 4°C). After centrifugation, 40 µL of the supernatant was dispensed into a vial and measured by LC-MS/MS.

Note that, the Whatman FTA DMPK-A Card may be hereinafter abbreviated as DMPK-A card. QIAcard FTA DMPK-B Card may be hereinafter abbreviated as DMPK-B card. QIAcard FTA DMPK-C Card may be hereinafter abbreviated as DMPK-C card. Further, Whatman 903 protein saver card may be hereinafter abbreviated as 903 card.

### LC-MS/MS measurement conditions

**[Table 5]**

| | | |
|---|---|---|
| Device | MS | Triple quadrupole mass spectrometer 4000 QTRAP (AB Sciex) |
| | LC | Ultra-high performance liquid chromatograph NexeraX2 (Shimadzu Corporation) |
| Column | | Atlantis Hilic (4.6 mm × 50 mm, 5 µm) (Waters) |
| Column temperature | | 50°C |
| Injection amount | | 3 to 5 µL |
| Flow rate | | 1.0 mL/min |
| Auto-sampler temperature | | 4°C |
| Mobile phase A | | 10 mM ammonium formate water containing 0.05% formic acid |
| Mobile phase B | | Acetonitrile |
| Washings 1 | | Acetonitrile:water (9:1, v/v) |
| Washings 2 | | Acetonitrile: Mobile phase A (97:3, v/v) |
| Ion Source | | Turbo Spray |
| Polarity | | Positive |

**[Table 6]**

| | Time (min) | B (%) |
|---|---|---|
| Gradient conditions | Initial | 80 |
| | 1.00 | 80 |
| | 3.00 | 40 |
| | 4.50 | 40 |
| | 4.60 | 80 |
| | 7.00 | 80 |

**[Table 7]**

| | Compound | Q1 | Q3 |
|---|---|---|---|
| Q1/Q3 | NAD | 664.056 | 136.1 |
| | NAD-d4 | 668.198 | 136.1 |
| | NMN | 335.098 | 123.1 |
| | NMN-d4 | 339.139 | 126.9 |

### Experimental result

The above experimental results are shown in Tables 8 to 11.

**[Table 8]**

| Quantifying target substance | Item | Filter paper: none | Filter paper: DMPK-A card | | | | | |
|---|---|---|---|---|---|---|---|---|
| | | Storage for 0 days | Chilled storage for 2 days | | Chilled storage for 2 weeks | | Chilled storage for 1 month | |
| | | Concentration (µM) | Concentration (µM) | Storage stability (%) | Concentration (µM) | Storage stability (%) | Concentration (µM) | Storage stability (%) |
| NAD+ | Mean | 31.89 | 24.05 | Good: 81.5 | 24.37 | Good: 79.1 | 20.69 | Good: 71.8 |
| | Standard deviation | 6.43 | 5.82 | 10.9 | 4.16 | 12.5 | 4.97 | 20.9 |
| | Number of cases | 24 | 4 | | 18 | | 4 | |
| NMN | Mean | 1.85 | 1.10 | Good: 73.1 | 1.37 | Good: 80.6 | 1.38 | Excellent: 99.9 |
| | Standard deviation | 0.43 | 0.15 | 20.2 | 0.32 | 18.4 | 0.38 | 41.9 |
| | Number of cases | 24 | 4 | | 18 | | 4(1) | |
| *Storage stability = Concentration (storage period x days) / Concentration (storage period 0 days) × 100 | | | | | | | | |
| *Evaluation = Excellent (>_85%), Good (>_70%), Average (>_50%), Poor (<50%) | | | | | | | | |
| *The number in parentheses in the row of Number of cases is the number excluded due to BLQ. BLQ= Below Limit of Quantitation | | | | | | | | |

**[Table 9]**

| Quantifying target substance | Item | Filter paper: DMPK-B card | | | | | |
|---|---|---|---|---|---|---|---|
| | | Chilled storage for 2 days | | Chilled storage for 2 weeks | | Chilled storage for 1 month | |
| | | Concentration (µM) | Storage stability (%) | Concentration (µM) | Storage stability (%) | Concentration (µM) | Storage stability (%) |
| NAD+ | Mean | 23.75 | Good: 80.7 | 27.43 | Excellent: 87.8 | 24.55 | Good: 79.8 |
| | Standard deviation | 5.43 | 11.0 | 5.05 | 11.2 | 4.92 | 13.9 |
| | Number of cases | 4 | | 23 | | 9 | |
| NMN | Mean | 1.41 | Excellent: 95.4 | 1.62 | Excellent: 93.6 | 1.61 | Good: 78.6 |
| | Standard deviation | 0.13 | 33.1 | 0.33 | 38.1 | 0.58 | 40.2 |
| | Number of cases | 4 | | 23(1) | | 9 (3) | |
| *Storage stability = Concentration (storage period x days) / Concentration (storage period 0 days) × 100 | | | | | | | |
| *Evaluation = Excellent (>_85%), Good (>_70%), Average (>_50%), Poor (<50%) | | | | | | | |
| *The number in parentheses in the row of Number of cases is the number excluded due to BLQ. | | | | | | | |

**[Table 10]**

| Quantifying target substance | Item | Filter paper: DMPK-C card | | | | | |
|---|---|---|---|---|---|---|---|
| | | Chilled storage for 2 days | | Chilled storage for 2 weeks | | Chilled storage for 1 month | |
| | | Concentration (µM) | Storage stability (%) | Concentration (µM) | Storage stability (%) | Concentration (µM) | Storage stability (%) |
| NAD+ | Mean | 22.02 | Good: 70.1 | 20.88 | Average: 66.6 | 16.74 | Average: 54.3 |
| | Standard deviation | 5.66 | 12.2 | 5.59 | 16.3 | 2.52 | 8.4 |
| | Number of cases | 5 | | 19 | | 5 | |
| NMN | Mean | 1.04 | Average: 68.4 | 0.91 | Average: 52.9 | BLQ | NC |
| | Standard deviation | 0.31 | 19.3 | 0.19 | 15.3 | NC | NC |
| | Number of cases | 5 | | 19(3) | | 5 (3) | |
| *Storage stability = Concentration (storage period x days) / Concentration (storage period 0 days) × 100 | | | | | | | |
| *Evaluation = Excellent (>_85%), Good (>_70%), Average (>_50%), Poor (<50%) | | | | | | | |
| *The number in parentheses in the row of Number of cases is the number excluded due to BLQ. | | | | | | | |

**[Table 11]**

| Quantifying target substance | Item | Filter paper: 903 card | | | | | |
|---|---|---|---|---|---|---|---|
| | | Chilled storage for 2 days | | Chilled storage for 2 weeks | | Chilled storage for 1 month | |
| | | Concentration (µM) | Storage stability (%) | Concentration (µM) | Storage stability (%) | Concentration (µM) | Storage stability (%) |
| NAD+ | Mean | 23.95 | Good: 74.1 | 24.33 | Good: 77.2 | 26.23 | Good: 82.6 |
| | Standard deviation | 6.75 | 10.5 | 5.53 | 12.6 | 4.27 | 11.0 |
| | Number of cases | 5 | | 14 | | 5 | |
| NMN | Mean | 1.10 | Average: 60.1 | 1.04 | Average: 64.5 | 1.02 | 56.3 |
| | Standard deviation | 0.14 | 9.3 | 0.28 | 29.6 | 0.27 | 16.8 |
| | Number of cases | 5 | | 14 (1) | | 5 | |
| *Storage stability = Concentration (storage period x days) / Concentration (storage period 0 days) × 100 | | | | | | | |
| *Evaluation = Excellent (≥85%), Good (≥70%), Average (≥50%), Poor (<50%) | | | | | | | |
| *The number in parentheses in the row of Number of cases is the number excluded due to BLQ. | | | | | | | |

As is clear from Tables 8 and 9 above, regarding NAD+, when using DMPK-A card, the NAD+ concentrations after storage for 2 days, 2 weeks, and 1 month were 81.5%, 79.1%, and 71.8% of the initial value (0 days), respectively, indicating good chilled storage stability. In addition, when using DMPK-B card, the NAD+ concentrations after storage for 2 days, 2 weeks, and 1 month were 80.7%, 87.8%, and 79.8% of the initial value (0 days), respectively, indicating chilled storage stability even better than DMPK-A card.

In contrast, as is clear from Table 10 above, when using DMPK-C card, the NAD+ concentrations after storage for 2 days, 2 weeks, and 1 month were 70.1% and 66.6%, and 54.3% of the initial value (0 days), respectively, and as is clear from Table 11 above, when using 903 card, the NAD+ concentrations after storage for 2 days, 2 weeks, and 1 month were 74.1%, 77.2%, and 82.6%% of the initial value (0 days), respectively. From these results, regarding NAD+, DMPK-A card and B card (especially DMPK-B card) are found to be superior in terms of chilled storage stability on the average throughout each period compared with DMPK-C card and 903 card.

In addition, regarding NMN, as is clear from Table 8 above, when using DMPK-A card, the NMN concentrations after storage for 2 days, 2 weeks, and 1 month were 73.1%, 80.6%, and 99.9% of the initial value (0 days), respectively, indicating good chilled storage stability. In addition, as is clear from Table 9 above, when using DMPK-B card, the NMN concentrations after storage for 2 days, 2 weeks, and 1 month were 95.4% and 93.6%, and 78.6% of the initial value (0 days), respectively, also indicating good storage stability comparable to that of DMPK-A card.

In contrast, as is clear from Table 10 above, when using DMPK-C card, the NMN concentrations after storage for 2 days, 2 weeks, and 1 month were 68.4%, 52.9%, and NC of the initial value (0 days), respectively, and as is clear from Table 11 above, when using 903 card, the NMN concentrations after storage for 2 days, 2 weeks, and 1 month were 60.1%, 64.5%, and 56.3% of the initial value (0 days), respectively. From these results, also regarding NMN, DMPK-A card and B card were found to be superior in terms of chilled storage stability compared with DMPK-C card and 903 card.

Considering the above results, DMPK-A card and B card were treated with chemical agents, whereas DMPK-C card and 903 card were not treated with chemical agents (Am. J. Trop. Med. Hyg., 99(2), 2018, pp. 256-265, or Bioanalysis, 2013, 5, 2613-30, etc.).

Therefore, the presence or absence of treatment with the chemical agent and the type thereof inferably contribute to the chilled storage stability.

Note that, regarding the above chemical agent, that of DMPK-A card is a radical inhibitor (sodium dodecyl sulfate), and that of DMPK-B card is a chaotropic agent (guanidine thiocyanate) (Am. J. Trop. Med. Hyg., 99(2), 2018, pp. 256-265, or Bioanalysis, 2013, 5, 2613-30, etc.).

### Example 3: Optimization study of filter paper based on the viewpoint of room temperature storage stability

A filter paper, to which blood derived from a test subject is added (i.e., corresponding to a filter paper in Step 1 of the present invention), was searched for the optimal one for quantifying NAD+ and NMN when the filter paper with added blood derived from the test subject is stored at room temperature for a certain period of time.

The specific experimental method is as follows.

### Experimental method

A part of the blood spotted-filter paper prepared in Example 2 was sealed in a moisture absorption standard pouch (Moisture absorption-kun (registered trademark)/Maruto Sangyo Co., Ltd.) and then stored at room temperature. After storage at room temperature for 2 days, 1 week (7 days), or 2 weeks (15 days), the blood-spotted areas were cut out and collected in a 2 mL microtube. After performing the same treatment as in Example 2, measurement by LC-MS/MS was performed under the same conditions as in Example 2.

### Experimental result

The above experimental results are shown in Tables 12 to 15.

**[Table 12]**

| Quantifying target substance | Item | Filter paper: none | Filter paper: DMPK-A card | | | |
|---|---|---|---|---|---|---|
| | | Storage for 0 days | Room temperature storage for 2 days | | Room temperature storage for 2 weeks | |
| | | Concentration (µM) | Concentration (µM) | Storage stability (%) | Concentration (µM) | Storage stability (%) |
| NAD+ | Mean | 31.95 | 21.71 | Good: 71.6 | 17.34 | Average: 51.6 |
| | Standard deviation | 6.15 | 4.08 | 7.7 | 2.94 | 5.6 |
| | Number of cases | 19 | 8 | | 5 | |
| NMN | Mean | 1.93 | 1.95 | Excellent: 122.7 | 2.09 | Excellent: 124.2 |
| | Standard deviation | 0.42 | 0.49 | 46.0 | 1.15 | 49.1 |
| | Number of cases | 19 | 8 | | 5 | |
| *Storage stability = Concentration (storage period x days) / Concentration (storage period 0 days) × 100 | | | | | | |
| *Evaluation = Excellent (≥85%), Good (≥70%), Average (≥50%), Poor (<50%) | | | | | | |

**[Table 13]**

| Quantifying target substance | Item | Filter paper: DMPK-B card | | | | | |
|---|---|---|---|---|---|---|---|
| | | Room temperature storage for 2 days | | Room temperature storage for 1 week | | Room temperature storage for 2 weeks | |
| | | Concentration (µM) | Storage stability (%) | Concentration (µM) | Storage stability (%) | Concentration (µM) | Storage stability (%) |
| NAD+ | Mean | 25.47 | Good: 82.0 | 26.95 | Excellent: 87.2 | 22.65 | Average: 67.6 |
| | Standard deviation | 4.91 | 9.9 | 4.64 | 21.4 | 3.16 | 4.6 |
| | Number of cases | 13 | | 7 | | 9 | |
| NMN | Mean | 1.71 | Excellent: 96.5 | 1.39 | Average: 69.1 | 1.17 | Average: 66.6 |
| | Standard deviation | 0.34 | 40.4 | 0.31 | 28.3 | 0.46 | 39.6 |
| | Number of cases | 13 | | 7 | | 9 (2) | |
| *Storage stability = Concentration (storage period x days) / Concentration (storage period 0 days) × 100 | | | | | | | |
| *Evaluation = Excellent (≥85%), Good (≥70%), Average (≥50%), Poor (<50%) | | | | | | | |
| *The number in parentheses in the row of Number of cases is the number excluded due to BLQ. | | | | | | | |

**[Table 14]**

| Quantifying target substance | Item | Filter paper: DMPK-C card | | | |
|---|---|---|---|---|---|
| | | Room temperature storage for 2 days | | Room temperature storage for 2 weeks | |
| | | Concentration (µM) | Storage stability (%) | Concentration (µM) | Storage stability (%) |
| NAD+ | Mean | 17.23 | Average: 55.9 | 17.02 | Average: 50.2 |
| | Standard deviation | 4.53 | 7.1 | 4.35 | 7.9 |
| | Number of cases | 7 | | 5 | |
| NMN | Mean | BLQ | NC | BLQ | NC |
| | Standard deviation | NC | NC | NC | NC |
| | Number of cases | 7(4) | | 5 (5) | |
| *Storage stability = Concentration (storage period x days) / Concentration (storage period 0 days) × 100 | | | | | |
| *Evaluation = Excellent (≥85%), Good (≥70%), Average (≥50%), Poor (<50%) | | | | | |
| *The number in parentheses in the row of Number of cases is the number excluded due to BLQ. | | | | | |

**[Table 15]**

| Quantifying target substance | Item | Filter paper: 903 card | | | |
|---|---|---|---|---|---|
| | | Room temperature storage for 2 days | | Room temperature storage for 2 weeks | |
| | | Concentration (µM) | Storage stability (%) | Concentration (µM) | Storage stability (%) |
| NAD+ | Mean | 20.63 | Average: 66.2 | 17.15 | Average: 50.3 |
| | Standard deviation | 6.25 | 7.6 | 4.44 | 6.4 |
| | Number of cases | 7 | | 5 | |
| NMN | Mean | 1.38 | Good: 79.8 | BLQ | NC |
| | Standard deviation | 0.21 | 15.6 | NC | NC |
| | Number of cases | 7(1) | | 5 (5) | |
| *Storage stability = Concentration (storage period x days) / Concentration (storage period 0 days) × 100 | | | | | |
| *Evaluation = Excellent (≥85%), Good (≥70%), Average (≥50%), Poor (<50%) | | | | | |
| *The number in parentheses in the row of Number of cases is the number excluded due to BLQ. | | | | | |

As is clear from Table 12 above, regarding NAD+, when using DMPK-A card, the NAD+ concentrations after storage for 2 days and 2 weeks were 71.6% and 51.6% of the initial value (0 days), respectively, indicating good storage stability. In addition, as is clear from Table 13 above, when using DMPK-B card, the NAD+ concentrations after storage for 2 days, 1 week, and 2 weeks were 82.0% and 87.2%, and 67.6% of the initial value (0 days), respectively, indicating room temperature storage stability even better than when using DMPK-A card.

In contrast, as is clear from Table 14 above, when using DMPK-C card, the NAD+ concentrations after storage for 2 days and 2 weeks were 55.9% and 50.2% of the initial value (0 days), respectively, and as is clear from Table 15 above, when using 903 card, the NAD+ concentrations after storage for 2 days and 2 weeks were 66.2% and 50.3% of the initial value (0 days), respectively. From these results, regarding NAD+, DMPK-A card and B card were found to be superior in terms of room temperature storage stability compared with DMPK-C card and 903 card.

In addition, regarding NMN, as is clear from Tables 12 and 13 above, although there exist some fluctuations in the values, storage stability will not be largely impaired when using DMPK-A card and B card, indicating the quantifiability of NMN.

In contrast, as is clear from Table 14 above, when using DMPK-C card, the NMN concentrations after storage for 2 days and 2 weeks were unable to be quantified. In addition, as is clear from Table 15 above, when using 903 card, although the NMN concentration after storage for 2 days was 79.8% of the initial value (0 days), that of after storage for 2 weeks was unable to be quantified. From these results, also regarding NMN, DMPK-A card and B card were found to be superior in terms of room temperature storage stability compared with DMPK-C card and 903 card.

Considering the above results, as in Example 2, the results are inferably caused by the presence or absence of treatment with a chemical agent and the type thereof.

### Example 4: Optimization study of filter paper based on the viewpoint of extraction efficiency

A filter paper, to which blood derived from a test subject is added (i.e., corresponding to a filter paper in Step 1 of the present invention), was searched for the optimal one for quantifying NAD+ and NMN from the viewpoint of extraction efficiency of NAD+ and NMN.

The specific experimental method is as follows.

### Experimental method

The extraction efficiency of NAD+ and NMN was evaluated for using and not using filter paper. The method for preparing the sample without filter paper is the same as in Example 2.

In contrast, the method for preparing the sample with a filter paper is also the same as the method in Example 2. That is, 4 types of filter papers (Whatman FTA DMPK-A Card/GE healthcare, QIAcard FTA DMPK-B Card/QIAGEN, QIAcard FTA DMPK-C Card/QIAGEN, Whatman 903 Protein saver card/Cytiva) were each spotted with 5 µL of blood, subjected to various treatments similar to those in Example 2, and then without chilled or room temperature storage for a certain period of time as in Examples 2 and 3, were measured using LC-MS/MS under the same conditions as in Example 2.

### Experimental result

The above experimental results are shown in Tables 16 and 17.

**[Table 16]**

| Quantifying target substance | Item | Filter paper: none | Filter paper: DMPK-A card | | Filter paper: DMPK-B card | |
|---|---|---|---|---|---|---|
| | | Concentration (µM) | Concentration (µM) | Extraction efficiency (%) | Concentration (µM) | Extraction efficiency (%) |
| NAD+ | Mean | 31.81 | 29.65 | Excellent: 95.1 | 28.75 | Excellent: 90.9 |
| | Standard deviation | 5.80 | 4.64 | 11.2 | 5.44 | 11.4 |
| | Number of cases | 14 | 9 | | 14 | |
| NMN | Mean | 1.96 | 2.01 | Excellent: 125.6 | 1.62 | Good: 87.7 |
| | Standard deviation | 0.47 | 0.57 | 61.8 | 0.46 | 36.9 |
| | Number of cases | 14 | 9 | | 14 | |
| *Extraction efficiency = Concentration (with filter paper) / Concentration (without filter paper) × 100 | | | | | | |
| *Evaluation = Excellent (≥90%), Good (≥85%), Average (≥75%), Poor (<75%) | | | | | | |

**[Table 17]**

| Quantifying target substance | Item | Filter paper: DMPK-C card | | Filter paper: 903 card | |
|---|---|---|---|---|---|
| | | Concentration (µM) | Extraction efficiency (%) | Concentration (µM) | Extraction efficiency (%) |
| NAD+ | Mean | 22.44 | Poor: 72.9 | 24.47 | Average: 77.2 |
| | Standard deviation | 4.41 | 18.1 | 8.23 | 19.0 |
| | Number of cases | 9 | | 9 | |
| NMN | Mean | 1.37 | Good: 87.9 | 1.20 | Poor: 73.2 |
| | Standard deviation | 0.69 | 58.6 | 0.36 | 25.2 |
| | Number of cases | 9 | | 9(1) | |
| *Extraction efficiency = Concentration (with filter paper) / Concentration (without filter paper) × 100 | | | | | |
| *Evaluation = Excellent (≥90%), Good (≥85%), Average (≥75%), Poor (<75%) | | | | | |
| *The number in parentheses in the row of Number of cases is the number excluded due to BLQ. | | | | | |

As is clear from Table 16 above, regarding NAD+, when using DMPK-A card and B card, the extraction efficiencies of NAD+ from the filter paper (calculated based on the concentration value without filter paper) were 95.1% and 90.9%, respectively, indicating good extraction efficiency.

In contrast, as is clear from Table 17 above, when using DMPK-C card and 903 card, the extraction efficiencies of NAD+ from the filter paper were 72.9% and 77.2%, respectively. From these results, regarding NAD+, DMPK-A card and B card were found to be superior in terms of extraction efficiency compared with DMPK-C and 903 cards.

In addition, regarding NMN, as is clear from Table 16 above, when using DMPK-A card and B card, the extraction efficiencies of NMN from filter paper (calculated based on the concentration value without filter paper) were 125.6% and 87.7%, respectively, indicating good extraction efficiency.

In contrast, as is clear from Table 17 above, when using DMPK-C card and 903 card, the extraction efficiencies of the filter paper were 87.9% and 73.2%, respectively. From these results, also regarding NAD+, DMPK-A card and B card were found to be superior in terms of extraction efficiency compared with DMPK-C card and 903 card.

An additional remark to the results of Examples 2 to 4 above is as follows: Since the DBS method as in the present invention is often performed commonly on the premise of including self-collection, the step of adding blood derived from a test subject to a filter paper and the step of quantifying a quantifying target substance contained in the filter paper are often performed at physically or temporally different stages. More specifically, the step of adding blood to a filter paper is performed, for example, by the test subject (e.g., a human) at home or the like. Thereafter, the filter paper is transported to an institution (testing institution, medical institution, etc.) that has a mass spectrometer such as LC-MS/MS, and the step of quantifying the quantifying target substance is performed at the institution. Therefore, when performing the DBS method, the filter paper is required to be stored at room temperature or under chilling for a certain period of time (for example, from several days to about one month). In addition, during storage of the filter paper, there is required the prevention of decomposition of the quantifying target substance. Therefore, the filter paper used in the DBS method is required to have excellent storage stability for the quantifying target substance.

From Examples 2 to 4 above, the DMPK-A card and B card (i.e., corresponding to a filter paper in Step 1 of the present invention) was shown to be able to satisfy the above properties. That is, these cards were found to be the optimal filter paper for the quantitative method using the DBS method for NAD+ and NMN.

### Example 5: Study on storage stability of extract solution

A filter paper with added blood derived from a test subj ect is brought into contact with a solvent (extraction buffer) to obtain the solvent after contact (hereinafter may be abbreviated as extract solution), which storage stability was investigated.

The specific method is as follows.

### Experimental method

A part of the blood extract solution (100% water) from the filter paper obtained and stored in Example 2 (15 days of chilled storage) and a part of the solution for LC-MS/MS measurement (water containing 67% acetonitrile) were frozen-stored. After 71 days of frozen storage, the supernatant of the former obtained by adding twice the amount of acetonitrile thereto, mixing and centrifuging (24,250 ×g, 10 minutes, 4°C), and the supernatant of the latter obtained by centrifuging (24,250 ×g, 10 minutes, 4°C) the solution after storage were both measured by LC-MS/MS under the same conditions as in Example 2.

### Experimental result

The above experimental results are shown in Tables 18 to 21.

**[Table 18]**

| Quantifying target substance | Item | Filter paper: none (Storage for 0 days) | Composition of extract solution: 100% water (extraction buffer state) | | | |
|---|---|---|---|---|---|---|
| | | | Filter paper: DMPK-A card | | Filter paper: DMPK-B card | |
| | | Concentration (µM) | Concentration (µM) | Storage stability (%) | Concentration (µM) | Storage stability (%) |
| NAD+ | Mean | 28.41 | 41.44 | 145.8 | 26.37 | 93.1 |
| | Standard deviation | 2.41 | 5.24 | 12.5 | 2.39 | 8.4 |
| | Number of cases | 4 | 4 | | 4 | |
| *Filter paper storage period = 15 days (chilled storage), extract solution storage period: 71 days (frozen) | | | | | | |
| *Storage stability = Concentration (storage period x days) / Concentration (storage period 0 days) × 100 | | | | | | |

**[Table 19]**

| Quantifying target substance | Item | Composition of extract solution: 100% water (extraction buffer state) | | | |
|---|---|---|---|---|---|
| | | Filter paper: DMPK-C card | | Filter paper: 903 card | |
| | | Concentration (µM) | Storage stability (%) | Concentration (µM) | Storage stability (%) |
| NAD+ | Mean | 58.05 | 200.6 | 75.50 | 266.3 |
| | Standard deviation | 21.28 | 62.0 | 17.83 | 59.6 |
| | Number of cases | 4 | | 4 | |
| *Filter paper storage period = 15 days (chilled storage), extract solution storage period: 71 days (frozen) | | | | | |
| *Storage stability = Concentration (storage period x days) / Concentration (storage period 0 days) × 100 | | | | | |

**[Table 20]**

| Quantifying target substance | Item | Filter paper: none (Storage for 0 days) | Composition of extract solution: twice the amount of acetonitrile added to extraction buffer | | | |
|---|---|---|---|---|---|---|
| | | | Filter paper: DMPK-A card | | Filter paper: DMPK-B card | |
| | | Concentration (µM) | Concentration (µM) | Storage stability (%) | Concentration (µM) | Storage stability (%) |
| NAD+ | Mean | 28.41 | 25.37 | 89.6 | 28.14 | 100.0 |
| | Standard deviation | 2.41 | 1.83 | 7.1 | 2.62 | 16.1 |
| | Number of cases | 4 | 4 | | 4 | |
| *Filter paper storage period = 15 days (chilled storage), extract solution storage period: 71 days (frozen) | | | | | | |
| *Storage stability = Concentration (storage period x days) / Concentration (storage period 0 days) × 100 | | | | | | |

**[Table 21]**

| Quantifying target substance | Item | Composition of extract solution: twice the amount of acetonitrile added to extraction buffer | | | |
|---|---|---|---|---|---|
| | | Filter paper: DMPK-C card | | Filter paper: 903 card | |
| | | Concentration (µM) | Storage stability (%) | Concentration (µM) | Storage stability (%) |
| NAD+ | Mean | 21.10 | 73.6 | 26.40 | 92.1 |
| | Standard deviation | 5.24 | 12.6 | 6.31 | 15.1 |
| | Number of cases | 4 | | 4 | |
| *Filter paper storage period = 15 days (chilled storage), extract solution storage period: 71 days (frozen) | | | | | |
| *Storage stability = Concentration (storage period × days) / Concentration (storage period 0 days) × 100 | | | | | |

As is clear from Tables 18 and 19 above, regarding extraction buffer for each filter paper, when stored in extraction buffer state of 100% water, the storage stability of NAD+ was 93.1% for the DMPK-B card, indicating good storage stability. The extraction buffer for DMPK-A card, C card, and 903 card exhibited significantly higher values of 145.8%, 200.6%, and 266.3%, respectively, compared with the initial value.

In contrast, as is clear from Tables 20 and 21, regarding extraction buffer for each filter paper, when stored in extraction buffer state with addition of acetonitrile, the storage stability of NAD+ for DMPK-A card, C card and 903 card was 89.6%, 73.6%, and 92.1%, respectively, indicating improved values from the extraction buffer state of 100% water.

Considering the above results, regarding DMPK-B card, NAD+ is inferably inactivated sufficiently at the time of extraction, resulting in being stable even in 100% water state. In contrast, regarding DMPK-A card, C card, and 903 card, inferably, the inactivation of NAD+ during extraction is insufficient, thus the value when stored in 100% water state is higher than the initial value due to the progress of the enzyme reaction, etc., which are inactivated by adding acetonitrile to achieve storage stability.

From the above results, followings were found: Although a certain amount of organic solvent such as acetonitrile is commonly added in this field for the storage stability of the extract solution, the addition of organic solvents such as acetonitrile to the extract solution is not necessary when using DMPK-B card, allowing easier quantification of NAD+. Further, an additional remark to this point is as follows: Elimination of the addition of a certain amount of organic solvent such as acetonitrile was suggestive of the possibility of using methods such as colorimetry and fluorescence methods other and simpler than the methods using mass spectrometers such as LC-MS/MS, for quantification of NAD+ in an extract solution. This possibility originates in the following: The addition of a certain amount of organic solvent such as acetonitrile causes problems of decreased sensitivity due to diluted NAD+ concentration in the sample and compatibility of acetonitrile with the reagents such as surfactants included in the kits of colorimetric and fluorescent method, which prevented the use of colorimetric and fluorescent methods.

### [Industrial Applicability]

The present invention can be utilized for the industry related to the quantitative method using the DBS method for NAD+ or NAD+ precursors such as NMN.

## Claims

1. A method for quantifying nicotinamide adenine dinucleotide (NAD+) or nicotinamide mononucleotide (NMN) in a test subject, the method comprising the following Steps 1 and 2:
Step 1 of bringing a filter paper, impregnated with a radical inhibitor or a chaotropic agent,
and with added blood derived from the test subject, into contact with a solvent;
Step 2 of quantifying the nicotinamide adenine dinucleotide (NAD+) or nicotinamide mononucleotide (NMN) in the solvent obtained in Step 1.

2. The method according to claim 1, wherein the method is for quantifying nicotinamide adenine dinucleotide (NAD+).

3. The method according to claim 1 or 2, wherein the filter paper is impregnated with a chaotropic agent.

4. The method according to claim 1 or 2, wherein the radical inhibitor contains a surfactant.

5. The method according to claim 4, wherein the surfactant is an anionic surfactant.

6. The method according to claim 5, wherein the anionic surfactant is sodium dodecyl sulfate.

7. The method according to claim 3, wherein the chaotropic agent contains a guanidine salt.

8. The method according to claim 7, wherein the guanidine salt is guanidine thiocyanate.

9. The method according to any one of claims 1 to 8, wherein the filter paper is made of cellulose.

10. The method according to any one of claims 1 to 9, wherein the solvent is water and/or acetonitrile.

11. The method according to claim 10, wherein the solvent is water.

12. The method according to any one of claims 1 to 11, wherein the quantifying in Step 2 is performed by a method using a mass spectrometer or a colorimetric method.

13. A kit for performing the method according to any one of claims 1 to 12, comprising: a blood sampling device and a filter paper impregnated with a radical inhibitor or a chaotropic agent.

14. A filter paper impregnated with a radical inhibitor or chaotropic agent for performing the method according to any one of claims 1 to 12.
